Europäisches Patentamt

European Patent Office

Office européen des brevets

(11) Numéro de publication: **0 248 701 B1**

# (12) FASCICULE DE BREVET EUROPEEN

(45) Date de publication de fascicule du brevet:
08.01.92

(51) Int. Cl.⁵: **A61K 7/48**

(21) Numéro de dépôt: 87401123.2

(22) Date de dépôt: **19.05.87**

(54) **Produit liquide pour le traitement esthétique de la peau et son procédé de fabrication.**

(30) Priorité: 30.05.86 FR 8607830

(43) Date de publication de la demande:
09.12.87 Bulletin 87/50

(45) Mention de la délivrance du brevet:
08.01.92 Bulletin 92/02

(84) Etats contractants désignés:
DE ES GB IT

(56) Documents cités:
EP-A- 0 022 046    CH-A- 246 132
DE-A- 2 141 819    FR-A- 1 142 928
FR-A- 2 077 787    FR-A- 2 165 856
FR-A- 2 191 878    FR-A- 2 260 326
FR-A- 2 573 651

(73) Titulaire: CLARINS
4, rue Berteaux-Dumas B.P. 174
F-92203 Neuilly-sur-Seine Cédex(FR)

(72) Inventeur: Courtin, Olivier
6 bis, rue de la Belle Feuille
F-92100 Boulogne(FR)

(74) Mandataire: Netter, André et al
Cabinet NETTER, 40, rue Vignon
F-75009 Paris(FR)

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**Description**

L'invention a pour objet un produit liquide pour le traitement esthétique de la peau et son procédé de fabrication.

On a proposé de très nombreux produits pour le traitement esthétique de la peau. Certains sont à base d'extrait végétal. L'extrait est, en principe, prévu pour exercer une action favorable d'une nature déterminée. Quelques-uns de ces produits sont sans odeur. D'autres ont, en soi, une odeur, quelquefois agréable.

Certains de ces produits comportent en addition des parfums, c'est-à-dire des substances naturelles ou artificielles qui leur confèrent une odeur agréable, mais qui n'ont, par eux-mêmes, aucune action bénéfique sur la peau. Les liquides ainsi obtenus, commercialisés notamment sous le nom d'eaux de toilette, sont plus plaisants à utiliser que ceux qui ne présentent pas une telle odeur agréable. Cependant, les consommateurs sont de plus en plus réticents à l'égard des additifs tels que les colorants et les parfums, destinés spécialement à influencer les caractéristiques sensorielles des produits. Certaines allergies ou réactions cutanées ont même été attribuées à la présence de parfums dans des produits destinés au traitement esthétique de la peau.

En particulier, DE-A-2 141 819 et FR-A-1 142 928 décrivent des produits cosmétiques, et EP-A-0 022 046 décrit un médicament pour le traitement des plaies, contenant, à côté d'extraits végétaux ayant une action bénéfique sur la peau, en particulier d'extraits de romarin, de thym et d'hamamélis, des additifs n'ayant pas une telle action, notamment des parfums.

L'invention a pour objets un produit, un procédé et une utilisation tels que définis dans les revendications.

Les extraits végétaux utilisés ont chacun une action bénéfique et sont choisis pour que leurs actions bénéfiques s'ajoutent les unes aux autres, permettant ainsi d'aboutir à un produit de traitement polyvalent, voire total.

Certains de ces extraits végétaux ont une odeur agréable, et, tout en respectant la condition de compatibilité avec les autres extraits végétaux au point de vue de leur action, conservent leur odeur, permettant ainsi d'obtenir un produit à odeur agréable sans adjonction d'un parfum n'ayant aucune action cutanée.

Le produit selon l'invention réalise simultanément le double objectif de traiter esthétiquement la peau de manière efficace et de parfumer celle-ci.

L'invention sélectionne les extraits végétaux odorants pour que leurs actions odorantes s'harmonisent entre elles. Les produits ainsi obtenus peuvent même, à cet égard, être considérés comme des produits d'aromathérapie.

Les extraits végétaux satisfont en outre à la condition, en ce qui concerne leur action odoriférante, de ne pas s'opposer, après que le produit ait été appliqué sur le corps, a l'action d'un parfum qui serait appliqué par la suite.

Cependant, dans bien des cas, on préfère utiliser le produit selon l'invention sans adjonction ultérieure de parfum, l'odeur qu'il dégage sur la peau permettant de s'abstenir d'un apport complémentaire de parfum. Un tel produit est alors particulièrement avantageux pour des personnes sujettes à des allergies et également pour l'utilisation dans des pays où l'intensité d'un parfum proprement dit est peu appréciée.

Le produit selon l'invention contient, à côté d'extraits végétaux à action cutanée bénéfique et à odeur agréable, des extraits végétaux inodores mais ayant une action bénéfique sur la peau. On peut ainsi aboutir à une large gamme d'effets sur la peau, profitant ici de la constatation, qui a été faite, que l'adjonction de tels extraits n'a pas d'effet négatif sur les extraits végétaux odorants, tant au point de vue de leur action sur la peau qu'au point de vue de leur odeur.

En particulier, les extraits inodores peuvent avoir une action hydratante que les extraits parfumés, qui sont en principe des huiles essentielles, sont incapables de procurer.

Les actions exercées sur la peau par un produit selon l'invention peuvent être diverses : astringente, tonifiante, affermissante, stimulante, tannante, rafraîchissante, hydratante, déodorante.

Ces actions peuvent être exercées individuellement ou en combinaison par les différents extraits, c'est-à-dire que chaque type d'action peut être dû à un seul extrait ou à la coopération de deux extraits, ou plus, une synergie pouvant être observée dans certains cas pour différents extraits.

Il est également possible, grâce au choix des extraits végétaux, d'obtenir un effet d'aromathérapie, c'est-à-dire une sensation de bien-être procurée par la respiration du produit pendant et/ou après son application sur la peau.

Les extraits végétaux, utilisés en solution hydro-alcoolique, sont avantageusement choisis dans le groupe comprenant les extraits de coriandre, de romarin, de thym, de citron, de gingembre, de cardamome, de noix de muscade, d'orange, de lavandin, d'éleuthérocoque, d'aloès, d'ispaghul, de prêle, d'hamamelis et de lierre.

Ces plantes sont connues pour leurs activités suivantes :

- Extraits odoriférants :
  . Coriandre (coriandrum satioum) : de son fruit est extraite une essence, soluble dans l'alcool, riche en d.linalol et contenant un peu de géraniol et de l.bornéol. Les études pharmacologiques effectuées sur cette huile essentielle mettent en évidence des effets stimulants.

. Romarin (rosmarinus officinalis) : des sommités fleuries est extraite une essence soluble dans l'alcool contenant de l'α-pinène aux propriétés tonifiantes, stimulantes, antiseptiques.

. Thym (thymus vulgaris) : les sommités fleuries et les feuilles contiennent une essence soluble dans l'alcool, renfermant du thymol. Son action est surtout tonifiante et bactéricide.

. Citron (citrus lomonum) : l'huile essentielle de citronnier extraite du zeste du fruit est soluble dans l'alcool et connue pour ses propriétés bactéricides et tonifiantes.

. Gingembre (zingiber officinale) : l'huile essentielle extraite du rhizome, riche en dérivés terpéniques, est appréciée pour ses propriétés stimulantes et tonifiantes.

. L'association d'huiles essentielles de cardamome, de noix muscade, d'orange et de lavandin renforce par action synergique les propriétés tonifiantes des essences ci-dessus mentionnées.

- Extraits inodores :

. L'éleuthérocoque (eleutherococcus senticosus) est utilisé compte tenu de la présence dans sa racine de principes actifs tonifiants et énergisants.

. L'aloès (aloe vera) contribue à maintenir le taux d'hydratatation et donc la souplesse et l'élasticité de l'épiderme.

. L'ispaghul (plantago ispaghula) dont on utilise les graines pour leurs propriétés hydratantes et anti-inflammatoires.

. La prêle (equisetum arveuse), dont les parties utilisées sont la tige et les feuilles, présente un intérêt particulier en raison de ses propriétés raffermissantes cutanées attribuées à la richesse en silicium de son extrait.

. Hamamelis (hamamelis virginiana) : par traitement, on extrait de la feuille des tannins galliques astringents et vasoconstricteurs.

. Lierre (hedera helix) : l'extrait de la feuille est doté de propriétés vasoconstrictrices et raffermissantes dues à sa richesse en saponosides.

Parmi ces extraits, ceux de coriandre, de romarin, de thym et de cardamome présentent une combinaison particulièrement intéressante de propriétés eudermiques et odoriférantes, et ceux d'éleuthérocoque, d'ispaghul et de prêle, qui sont inodores, présentent une combinaison particulièrement avantageuse de propriétés eudermiques. Les produits liquides selon l'invention contiennent donc au moins ces extraits.

Un mélange, objet de l'invention, peut également comprendre un ou des extraits de plantes odoriférants ci-après, dont les propriétés sont voisines de celles des extraits cités précédemment : basilic, patchouli, géranium, neroli, petit grain.

L'invention a également pour objet un procédé de fabrication d'un produit comme visé ci-dessus, selon lequel on met en macération des parties de plantes actives dans une solution hydro-alcoolique, après quoi on filtre. On élimine ainsi la fraction non soluble dans un mélange hydro-alcoolique.

On décrira maintenant un exemple de mise en oeuvre de l'invention.

On met en ccntact avec 1,5 kg d'un mélange hydro-alcoolique à 90° d'alcool des parties actives des plantes suivantes, dans les quantités ci-après :
- Coriandre 10 g - Citron 40 g
- Romarin 50 g - Cardamome 20 g
- Thym 20 g - Noix de muscade 10 g
- Orange 50 g - Aloès 40 g
- Lavandin 20 g - Ispaghul 40 g
- Eleuthérocoque 40 g - Prêle 40 g
- Gingembre 40 g - Lierre 40 g
- Hamamelis 40 g

Après macération pendant une dizaine de jours à une température de 15° C, sous agitation lente discontinue, on filtre le mélange sur filtre-presse à une basse température (environ 5° C).

Le produit obtenu se présente sous forme d'une solution fluide limpide, de parfum agréable. La proportion globale d'extraits végétaux dans le produit fini est de 6% en poids environ, l'alcool éthylique à 90° représentant le complément à 100 %.

Les principes aromatiques odoriférants contenus dans la préparation ont tous une action positive lorsqu'ils sont appliqués sur l'épiderme. Cette activité est complétée par l'effet des autres extraits végétaux, qui n'ont pas de rôle sur la fragrance du produit mais qui en renforcent les propriétés tonifiantes.

L'odeur d'un liquide selon l'invention, tout en étant agréable, est discrète et ne nuit pas à l'utilisation ultérieure, par la personne sur laquelle il a été appliqué, du parfum de son choix. Il ne risque pas non plus d'incommoder l'entourage de l'utilisateur, comme pourraient le faire certaines eaux de toilette parfumées artificiellement.

Les caractéristiques avantageuses de la lotion selon l'invention sont de nature à favoriser un emploi régulier de celle-ci, permettant, grâce à son action bénéfique sur la peau, et en particulier à son action raffermissante, d'améliorer dans le temps la fermeté de la peau, et par suite l'aspect du corps, et d'éviter l'apparition de défauts cutanés tels que les vergetures.

## Revendications

1. Produit liquide d'odeur agréable pour le traitement esthétique de la peau, caractérisé en ce qu'il est composé exclusivement d'extraits végétaux ayant une action cutanée bénéfique, en solution dans l'eau et dans l'éthanol, ces extraits comprenant au moins ceux de coriandre, de romarin, de thym, de cardamone, d'éleuthérocoque, d'ispaghul et de prêle.

2. Produit selon la revendication 1, caractérisé en ce qu'il comprend au moins un extrait choisi parmi ceux de citron, de gingembre, de noix de muscade, d'orange, de lavandin, d'aloès, d'hamamelis, de lierre, de basilic, de patchouli, de géranium, de neroli et de petit grain.

3. Produit selon l'une des revendications 1 et 2, caractérisé en ce que la proportion des extraits végétaux desdites plantes est de 6% en poids environ.

4. Procédé de fabrication d'un produit liquide selon l'une des revendications précédentes, caractérisé en ce qu'on met en macération des parties de plantes actives dans une solution eau-éthanol et qu'on filtre.

5. Procédé selon la revendication 4, caractérisé en ce qu'on met en macération les parties de plantes actives dans une solution hydro-alcoolique titrant 90°, et cela pendant une dizaine de jours à une température de 15°C, sous agitation lente discontinue, et en ce qu'on filtre sur filtre-presse à basse température de l'ordre de 5°C.

6. Procédé selon l'une des revendications 4 et 5, caractérisé en ce qu'on fait macérer ensemble toutes les parties de plantes dont le produit liquide à obtenir doit contenir les extraits, le produit final étant constitué par la solution obtenue après filtration sans additif supplémentaire.

7. Utilisation en tant que cosmétique d'un produit liquide selon l'une des revendications 1 à 3.

## Claims

1. Liquid product with a pleasant odour for the cosmetic treatment of skin, characterised in that it is composed exclusively of vegetable extracts having a beneficial skin action, in solution in water and in ethanol, these extracts comprising at least those of coriander, rosemary, thyme, cardamom, Siberian ginseng, ispaghul and horsetail.

2. Product according to claim 1, characterised in that it comprises at least one extract chosen from amongst those of lemon, ginger, nutmeg, orange, lavandin, aloe, witch hazel, ivy, basil, patchouli, geranium, neroli and petit grain.

3. Product according to one of claims 1 and 2, characterised in that the proportion of the vegetable extracts of the said plants is approximately 6% by weight.

4. Method of manufacturing a liquid product according to one of the preceding claims, characterised in that active parts of plants are steeped in a water/ethanol solution and in that filtration is carried out.

5. Method according to claim 4, characterised in that the active parts of plants are steeped in a water/alcohol solution (90° alcohol), for about ten days at a temperature of 15°C, with intermittent slow agitation, and in that filtration is carried out on à filter press at a low temperature of the order of 5°C.

6. Method according to one of claims 4 and 5, characterised in that all the parts of plants whose liquid product to be obtained is to contain the extracts are steeped together, the final product consisting of the solution obtained after filtration without any further additive.

7. Use as a cosmetic of a liquid product according to one of claims 1 to 3.

## Patentansprüche

1. Flüssige Zusammensetzung mit angenehmem Geruch für ästhetische Hautbehandlung, **dadurch gekennzeichnet,** daß sie ausschließlich aus pflanzlichen Extrakten, die eine pflegende Wirkung auf die Haut haben, in Wasser- und Äthanollösung zusammengesetzt ist, wobei in diesen Extrakten mindestens diejenigen von Koreander, Rosmarin, Thymian, Kardamon, plantago ispaghula (ispaghul) und Schachtelhalm enthalten sind.

2. Zusammensetzung nach Anspruch 1, **dadurch gekennzeichnet**, daß sie mindestens einen Extrakt der folgenden Pflanzen enthält: Zitrone, Ingwer, Muskatnuß, Orange, Lavendel, Aloe, Hamamelis, Efeu, Basilikum, Patschuli, Geranie, Orangenblüte und Petitgrain.

3. Zusammensetzung nach Anspruch 1 oder Anspruch 2, **dadurch gekennzeichnet,** daß der Gewichtsanteil der Pflanzenextrakte ungefähr 6 % beträgt.

4. Verfahren zur Herstellung der flüssigen Zusammensetzung gemäß einem der vorhergehenden Ansprüche, **dadurch gekennzeichnet,** daß die wirksamen Pflanzenteile in einer Wasser-Äthanol-Lösung eingeweicht werden und eine Filtration durchgeführt wird.

5. Verfahren nach Anspruch 4, **dadurch gekennzeichnet,** daß die wirksamen Pflanzenteile in einer hydroalkoholischen Lösung mit 90 % Alkoholanteil etwa zehn Tage lang bei einer Temperatur von 15° unter langsamem, zeitweisem Rühren ausgelaugt werden, und daß bei tiefer Temperatur von etwa 5° C mittels einer Filterpresse ein Filtrationsvorgang durchgeführt wird.

6. Verfahren nach Anspruch 4 oder Anspruch 5, **dadurch gekennzeichnet,** daß sämtliche Pflanzenteile, deren Extrakte die herzustellende flüssige Zusammensetzung enthalten soll, gemeinsam ausgelaugt werden und das Endprodukt aus der nach der Filtration erhaltenen Lösung ohne weitere Zusätze besteht.

7. Verwendung der Zusammensetzung nach einem der Ansprüche 1 bis 3 als Kosmetikum.